# EUROPEAN PATENT APPLICATION

(11) **EP 3 928 731 A1**
(43) Date of publication of application: **29.12.2021**
(21) Application number: 21181764.8
(22) Date of filing: 25.06.2021
(51) Int. Cl.: A61B 90/00, A61B 6/10, A61B 6/00, G21F 3/00

(54) **MICROBIAL AGENT PROTECTIVE DOME**

(30) Priority: 25.06.2020 EC SMU20034913 U
(71) Applicant: Pérez Martínez, Johann Stiven, 170156 Quito (EC)
(72) Inventor: Pérez Martínez, Johann Stiven, 170156 Quito (EC)
(74) Representative: Soler Lerma, Santiago

(57) **Abstract**

Protector against aerosols for use in the healthcare field that comprises a hemisphere made of transparent material, suitable for placing the patient's intervention zone under it. The protector comprises a support foot for the hemisphere as well as an anchoring member that allows the vertical, horizontal and pitch movement thereof. It may optionally incorporate a skirt to downwardly increase the cavity of the hemisphere.

## Description

The present invention refers to a protective, easy-to-move, versatile to operate and fully aseptic device adequate as a complement of biosecurity for dental clinics, hospitals, laboratories or places that require care and auxiliary protection for their professionals and work environment.

The dome-shaped transparent device is effective, versatile, easy to clean and reduces aerosol contamination by 95%.

The technical sector to which it belongs is that of sanitary elements and accessories.

### BACKGROUND

Protective screens have been known for a long time in the medical field, usually to protect the health care provider against possible infections and especially in the field of radiology as protection against radiation.

Examples of these are found in patent WO02/15198 referring to a vertical screen that protects against radiation.

We found more recent Chinese patents CN201912652, CN202313383, CN202477712, CN204542179, CN208677424 or, all of them referring to different types of screens against radiation.

German patent DE2714859 refers to a protective screen for certain parts of the patient's own body for protection against radiation.

Chinese patent CN21052111 refers to a protective screen with a transparent window at the front and opened by its side.

More recent is Spanish utility model ES1248355U referring to a screen for protection against aerosols comprising a sheet attached to a foot to which a skirt can be incorporated, or utility model ES1250044U referring to a large-sized transparent part to cover the width of a stretcher attached to a support.

None of the solutions indicated effectively protects against aerosols because, although it is an obstacle that hinders the direct route between the emitter and the receiver, it does not prevent aerosols from escaping from the sides and, due to their ability to remain in suspension for a long time, they eventually end up reaching the receiver.

### DESCRIPTION OF THE INVENTION

The invention proposed relates to a device that stands out by providing the healthcare professional, his patient and the work environment a high degree of asepsis since, due to its semi-spherical shape and its lower prolongation in the form of a cavity perimetrally defined by a laminated anti-fluid part, all the pathogens are isolated, allowing effective productivity and disinfection, as they do not have shaped angles.

Accordingly, the device comprises:
A dome made of transparent material, preferably an anti-fog PMMA with a percentage of methyl methacrylate over 90% made by thermoforming.

A fastening ring joined to the dome through easy-to-remove joining means and in turn attached to an anchoring organ that associates the ring to the support structure. This anchoring organ allows the pitch or oscillation of the dome being the anchoring organ the axis on which it oscillates.

A support structure, comprising a trunk and a foot, which supports the dome and is preferably made of grade 304 stainless steel.

This structure supports the anchoring organ which in turn supports the dome. The anchoring organ fastened to the trunk of the support structure allows the horizontal rotation of the dome and said trunk, preferably, acts as axis of the horizontal rotation of the dome.

The anchoring organ has means to be confined to a greater or lesser extent to the trunk, thus allowing vertical mobility of the dome.

All the parts that make it up can be removable, providing total disinfection and versatility for the execution of various health procedures. This structure comprises anchoring organs, a trunk and a preferably asymmetrical foot.

We will call asymmetrical a foot that has one of its supports greater than the others.

In a preferred embodiment, the device comprises a skirt made of a removable, anti-fluid, soft, laminated material, such as a fabric, arranged perimetrally so that it forms a cavity that prolongs the interior hollow of the dome.

The main advantage resulting from this invention is that the dome, being made by thermoforming of anti-fog, high-molecular-weight PMMA (methyl methacrylate in a percentage over 90%), manages to encapsulate and retain all pathogens, without distorting visibility or color.

The protective device thus made is easy to move and fully aseptic.

The transparent dome constitutes a hemispherical visor of 50cm in diameter, to which can be associated, perimetrally, a section of removable, anti-fluid, laminated fabric for disinfection and reuse, with a plurality of perforations, preferably 6 so that the user can introduce the arms and carry out the necessary operations.

The dome is supported by a stainless steel, preferably grade 304, structure with adjustable height, and mobility because it incorporates wheels with brake.

The device thus configured combines protection, vision and effective operability.

A number of drawings are provided for improved comprehension.

### BRIEF DESCRIPTION OF THE DRAWINGS

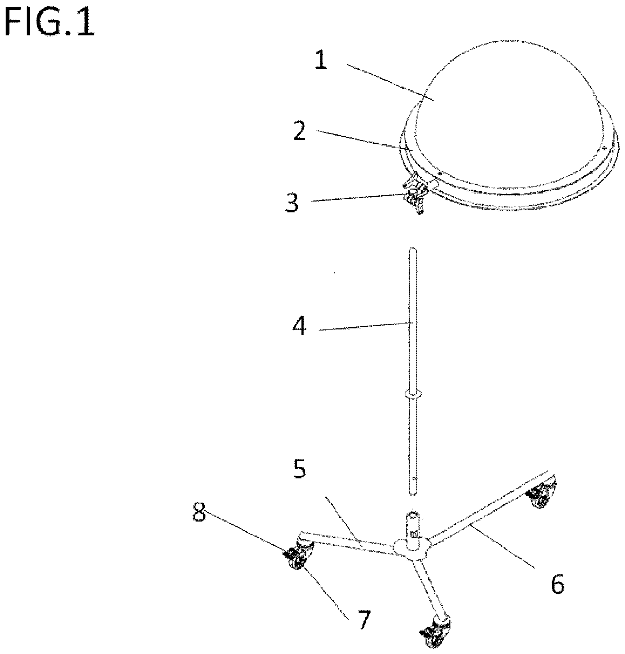

FIGURE 1 shows a blow-up of the main components of the device and so we find the dome (1) with its fastening ring (2) the anchoring organ (3) to the trunk (4) and the foot (5), in this case asymmetric, comprising three supports, one of them being a prolonged support (6) and wheels (7), preferably with brake (8). In this figure, the dome (1) is located in the vertical on the prolonged support (6).
FIGURE 2 shows the device to which the skirt (9) has been incorporated made of a fabric of anti-fluid laminated material that can be disposable. In the skirt there is a series of openings (10) in this case in the form of a buttonhole, suitable for introducing the hands and acting on the patient.

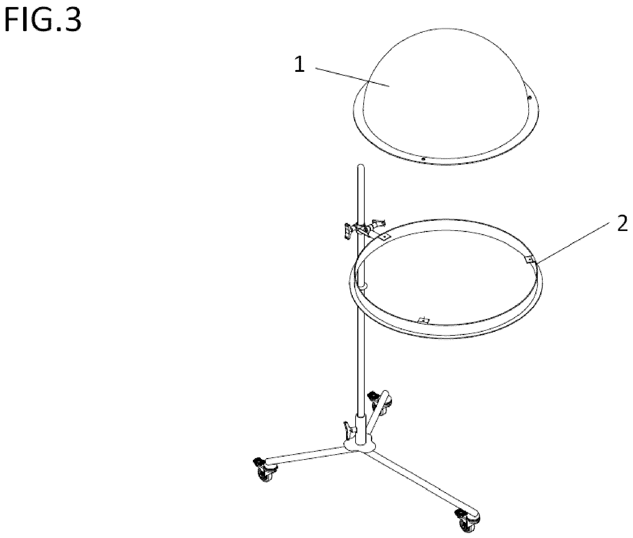

FIGURE 3 is a view of the device and a partial blow-up that shows the dome (1) separated from its fastening ring (2).
FIGURE 4 shows the device seen from the side where the dome (1) is located on the opposite side of the prolonged side (6).

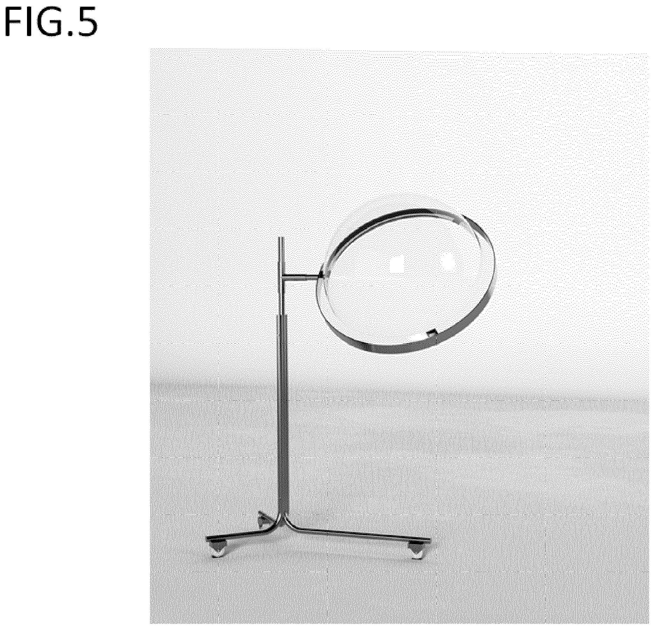

FIGURE 5 shows the device with the dome slightly tilted, as an example of the possibility of oscillation thereof.

### DESCRIPTION OF AN EMBODIMENT OF THE INVENTION

A way of carrying out the invention is described below with the express clarification that, since it is an example, it is not appropriate to assign to it an exclusive or limiting character of the scope of protection of the present invention, but simply a merely explanatory and illustrative intention of the basic line on which it is based.

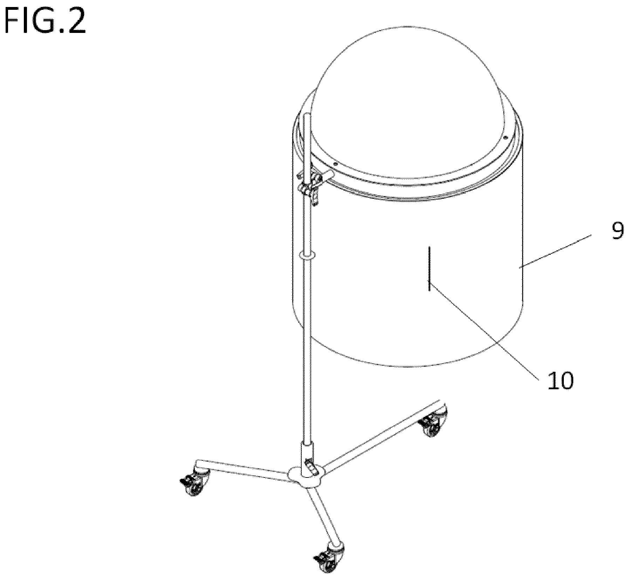

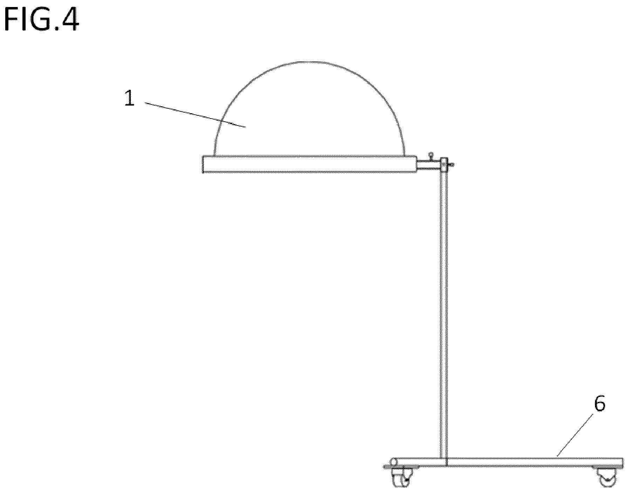

The device comprises:
1. A dome (1) that is hemispherical and therefore hygienic, since its curvature avoids any corners where a microbial agent accumulates, thus contributing to its asepsis. The dome is fastened by means of a removable fastening to a ring (2) that is associated with the anchoring organ (3) to secure the dome to the trunk (4) by means of a removable fastening, allowing, through means of snugness, the vertical mobility of the dome to adjust its required height on each occasion. The anchoring organ allows the horizontal rotation of the dome on the trunk, from the position shown for example in Figure 2 to that shown in Figure 4 and also allows the oscillation or pitch of the dome as shown in figure 6. This oscillation movement allows the dome to rotate 360°, making it easy to reverse its position and leave its opening at the top to facilitate cleaning operations.
2. A trunk (4) that supports the dome and joins the foot (5) with the dome (1). In the planned embodiment, the trunk comprises a tubular body with suitable means for anchoring to the foot (5) and unpin from it.
3. A foot (5) preferably asymmetrical comprising three supports, one of them being a prolonged support (6). This prolonged support considerably increases the support base of the device, giving it stability both in the position shown for example in Figure 2 to that shown in Figure 4.
4. A skirt (9) with an elastic upper contour, and 6 perforations for maneuvering, handling, suction and handpieces. It can be removed for autoclave disinfection and reuse, although it can also be disposable.

A high-molecular-weight acrylic sheet (methyl methacrylate in a percentage over 90%) is the basis of anti-fog thermoforming, alcohol-resistant, quaternary ammonium, and hydrogen peroxide for disinfection.

The dome is made of 3mm-thick, high-molecular- weight anti-fog PMMA with a light transmission rate of 92%, and high optical quality, and the loss of optical definition due to light ray scattering is only an average of 1%.

Its dimensions are based on studies of the patient's anatomy and operator's ergonomics, taking as optimal and most versatile a dome diameter of 50cm.

The dome is hemispherical because it is the geometry without edges of greater open base which facilitates the application on the patient and the work in its internal part.

In this way, through the combination of horizontal movement, oscillation and height adjustment, the device is completely versatile and can be adapted to any required circumstance.

In addition, the device is completely removable, which also facilitates the cleaning and disinfection operations of each of its parts.

In a preferred embodiment, the device is 120cm high.

In the manufacture of the device, right angles have been avoided to prevent the accumulation of pathologies.

## Claims

1. MICROBIAL AGENT PROTECTIVE DOME **characterized by** comprising:
A dome (1) made of anti-fog, high-molecular-weight PMMA.
A ring (2) that fastens the dome with a removable fastening.
An anchoring organ (3) that fastens the dome (1) to the trunk (5) through a removable fastening.
A vertical trunk (4) that attaches to the anchoring organ through a removable fastening.
A foot (5) joined to the trunk (4).

2. MICROBIAL AGENT PROTECTIVE DOME according to claim 1 **characterized by** further comprising a skirt (9) with a plurality of openings (10).

3. The MICROBIAL AGENT PROTECTIVE DOME according to claim 2 **characterized in that** the skirt (9) comprises an elastic strip on its top.

4. The MICROBIAL AGENT PROTECTIVE DOME according to claim 1 **characterized in that** the dome has a diameter of 50cm.

5. The MICROBIAL AGENT PROTECTIVE DOME according to claim 1 **characterized in that** the dome has a thickness of 3mm.

6. The MICROBIAL AGENT PROTECTIVE DOME according to claim 1 **characterized in that** the dome has horizontal movement on the trunk.

7. The MICROBIAL AGENT PROTECTIVE DOME according to claim 1 **characterized in that** the dome has oscillatory movement.

8. The MICROBIAL AGENT PROTECTIVE DOME according to claim 1 **characterized in that** the dome has vertical movement.

9. The MICROBIAL AGENT PROTECTIVE DOME according to claim 1 **characterized in that** the foot is asymmetrical.

10. The MICROBIAL AGENT PROTECTIVE DOME according to claim 1 **characterized in that** the PMMA comprises a percentage of over 90% of methyl methacrylate.
